# EUROPÄISCHE PATENTSCHRIFT

(11) **EP 4 205 636 B1**
(45) Veröffentlichungstag und Bekanntmachung des Hinweises auf die Patenterteilung: **24.07.2024**
(21) Anmeldenummer: 21218300.8
(22) Anmeldetag: 30.12.2021
(51) Int. Cl.: A61B 5/00, A61B 5/06

(54) **VORRICHTUNG MIT WUNDAUFLAGE, BILDAUFNAHMEMITTEL UND BEHANDLUNGSELEMENT**
DEVICE WITH WOUND DRESSING, IMAGE RECORDING MEANS AND TREATMENT ELEMENT
DISPOSITIF À PANSEMENT, MOYEN D'ENREGISTREMENT D'IMAGES ET ÉLÉMENT DE TRAITEMENT

(43) Veröffentlichungstag der Anmeldung: 05.07.2023
(73) Patentinhaber: Paul Hartmann AG, 89522 Heidenheim (DE)
(72) Erfinder: DEIBLER, Martin, 89542 Herbrechtingen (DE); CROIZAT, Pierre, 89542 Herbrechtingen (DE)
(74) Vertreter: Paul Hartmann AG Patents & Licensing

(56) Entgegenhaltungen:
- US-A1- 2010 100 160
- US-A1- 2020 188 180
- US-A1- 2020 289 347
- US-A1- 2020 297 244
- US-A1- 2020 352 501
- US-A1- 2021 268 276

## Beschreibung

Die vorliegende Erfindung betrifft eine Vorrichtung umfassend eine Wundauflage, welche einen therapeutischen Effekt auf die zu behandelnden Wunde ausüben kann, und ein Bildaufnahmemittel.

Wundabdeckungen, speziell selbstklebende Wundauflagen mit freisetzbaren aktiven Substanzen oder mit physikalischen Behandlungselementen wie beispielsweise einem Thermoelement, besitzen den Nachteil, dass die aktive Substanz bzw. die physikalische Behandlung aufgrund der festen Form der Wundauflage auch auf intakte Hautbereiche oder auf Bereiche der Wunde, welche keiner Behandlung bedürfen, einwirkt. An die Form der Wunde anpassbare aktive Wundkontaktschichten sind aufwändig in der Applikation und bergen bei der Anpassung ein zusätzliches Infektionsrisiko.

Die US2021268276 beschreibt eine Wundauflage, die mehrere Elektrodenpaare umfasst. Das Aktivieren der Elektrodenpaare generiert elektrische Stimuli, die die Wundheilung gezielt fördern.

Der Erfindung liegt die Aufgabe zu Grunde eine Vorrichtung bereitzustellen, die eine verbesserte Behandlung einer Wunde ermöglicht. Eine weitere Aufgabe liegt darin, eine Vorrichtung bereitzustellen, durch die eine ressourcenschonende Behandlung einer Wunde ermöglicht wird. Eine weitere Aufgabe der Erfindung liegt darin, eine Vorrichtung bereitzustellen, durch die eine präzise Behandlung derjenigen Bereiche der Wunde, welche tatsächlich einer Behandlung bedürfen, ermöglicht wird. Dabei sollte möglichst eine therapeutische Einwirkung auf die die Wunde umgebende intakte Haut vermieden werden.

Die Aufgaben werden durch eine Vorrichtung nach Anspruch 1 gelöst Kein Verfahren bildet Teil der Ansprüche.

Die anspruchsgemäße Vorrichtung ermöglicht einerseits, innerhalb einer zu behandelnden Wunde einen therapeutischen Effekt selektiv nur auf einen Teilbereich der zu behandelnden Wunde auszuüben. Anderseits ermöglich es die anspruchsgemäße Vorrichtung, innerhalb einer Wundstelle, welche die zu behandelnde Wunde und die die Wunde umgebende Haut umfasst, nur auf die eigentliche Wunde einen therapeutischen Effekt auszuüben, d.h. die die Wunde umgebende unversehrte Haut von der therapeutischen Einwirkung auszuschließen. Die in der Vorrichtung enthaltene Wundauflage umfasst eine bei Gebrauch der Wundauflage wundabseitig angeordnete Abdeckschicht und ein erstes Senderempfängerelement, welches Daten übertragen und empfangen kann. Die Wundauflage umfasst außerdem ein Behandlungselement, welches mindestens zwei unabhängig aktivierbare Bereiche aufweist. Mittels der mindestens zwei unabhängig aktivierbaren Bereiche kann entweder ein therapeutischer Effekt innerhalb der zu behandelnden Wunde nur auf einen Teilbereich der zu behandelnden Wunde ausgeübt werden oder ein therapeutischer Effekt innerhalb einer Wundstelle, welche die zu behandelnde Wunde und die die Wunde umgebende Haut umfasst, nur auf die Wunde ausgeübt werden.

Die Vorrichtung umfasst ebenfalls ein Bildaufnahmemittel. Das Bildaufnahmemittel umfasst eine digitale Kamera, einen ersten Prozessor und ein zweites Senderempfängerelement, welches dazu ausgestaltet ist Daten von dem ersten Senderempfängerelement zu empfangen und an das erste Senderempfängerelement zu senden.

In einer bevorzugten Ausführungsform ist das Behandlungselement bei Gebrauch der Wundauflage in direktem Kontakt mit dem Wundgrund.

In einer anderen bevorzugten Ausführungsform umfasst die Wundauflage eine Wundkontaktschicht. Die Wundkontaktschicht ist bei Gebrauch der Wundauflage wundseitig angeordnet. Falls eine Wundkontaktschicht vorgesehen ist, so sind die unabhängig aktivierbaren Bereiche bevorzugt in einer zu der Wundkontaktschicht parallelen Ebene angeordnet.

Jeder unabhängig aktivierbare Bereich hat keine gemeinsame Fläche mit seinen benachbarten Bereichen und kann einzeln aktiviert werden. Die unabhängig aktivierbaren Bereiche umfassen elektronische Bauteile, die in einer matrixförmigen Anordnung auf einem flexiblen Substrat wie beispielsweise Silikon aufgebracht oder eingebettet sind.

Die elektronischen Bauteile der unabhängig aktivierbaren Bereiche können beispielsweise Lichtleiter, Photodioden, , piezoelektrische Ultraschallemitter umfassen.

Falls die aktivierbaren Bereiche Photodioden umfassen, so können durch Wechselwirkung der Photonen mit der Wunde, der Wundauflage oder mit photoaktivierbaren Agenzien Behandlungseffekte erzielt werden. Die Behandlungseffekte können beispielsweise auf einer unmittelbaren lokalen Erhitzung, auf einer Sauerstoffradikalbildung oder auf Plasmonresonanzphänomenen mit lokal induzierter Wärmefreisetzung, die der Wundheilung förderlich ist, beruhen. Hierbei müssen Photodioden eingesetzt werden, welche Licht abstrahlen, das auf die gewünschte Behandlung abgestimmt ist.

Die Verwendung von Ultraschallarrays ermöglicht die Entfernung von Biofilmen oder bakteriellen Ansammlungen durch Mikro-Kavitation.

In einer bevorzugten Ausführungsform umfasst die Vorrichtung einen zweiten Prozessor, der eine Komponente der Wundauflage ist. Der zweite Prozessor kann die unabhängig aktivierbaren Bereiche des Behandlungselementes steuern. Bevorzug ist der zweite Prozessor ein Mikrocontroller.

Auf der Wundauflage befindet sich das erste Senderempfängerelement. Das erste Senderempfängerelement kommuniziert mit einem zweiten Senderempfängerelement. Bevorzugt soll dazu eine drahtlose Kommunikation mit einer Senderausgangsleistung von höchstens 1000 mW, bevorzugt von höchstens 100 mW und besonders bevorzugt von höchstens 10 mW verwendet werden. Besonders bevorzugt beruht die drahtlose Kommunikation dabei auf Bluetooth, Bluetooth ultra low energy oder WiFi. Es sind auch Datenübertragungssysteme wie Zigbee, Wibree oder andere Verfahren im Radiofrequenzbereich denkbar.

Die elektronischen Bauteile der unabhängig aktivierbaren Bereiche, des ersten Senderempfängerelements, sowie des optionalen zweiten Prozessors, können über eine Batterie, induktives Laden oder über Energy Harvesting mit Strom versorgt werden. Beim Energy Harvesting wird die in der Umgebung zur Verfügung stehende Energie in elektrische Energie umgewandelt. Die vorliegende Erfindung nutzt bevorzugt elektromagnetische Strahlung als Energiequelle. Besonders bevorzugt sind dabei Radiowellen, welche Frequenzen von 30 kHz bis zu 300 GHz besitzen. Anders als im Stand der Technik müssen die Radiowellen dabei vorzugsweise nicht oder nicht ausschließlich über ein speziell angepasstes Lesegerät erzeugt werden. Stattdessen können die in der Umgebung schon vorhandenen Radiowellen genutzt werden. Diese Radiowellen können beispielsweise von Funktürmen, Radaranlagen, WiFi-Netzen oder Bluetooth-Verbindungen stammen. Für WiFi Netze sind Radiowellen mit Frequenzen von 2,4 GHz und einer Leistung bis 100 mW, sowie mit Frequenzen von 5 GHz und einer Leistung bis 1 W von besonderem Interesse. Auf Mobilfunknetzen/GSM beruhende Radiowellen haben nach den GSM-Standards die Frequenzen 850 MHz, 900 MHz, 1800 MHz und 1900 MHz.

Die Wundauflage kommuniziert mit einem Bildaufnahmemittel, das mittels des zweiten Senderempfängerelements Daten von der Wundauflage empfängt und an die Wundauflage sendet. Unter Einsatz des Bildaufnahmemittels kann entweder die eigentliche Wunde innerhalb der Wundstelle oder innerhalb einer Wunde der zu behandelnde Bereich der Wunde bestimmt werden. Das zweite Senderempfängerelement kann dabei ebenso wie das erste Senderempfängerelement beispielsweise ein Bluetooth-Senderempfänger oder ein WiFi-Senderempfänger sein. Das Bildaufnahmemittel umfasst neben dem zweiten Senderempfängerelement, welches dazu ausgestaltet ist Daten von einem ersten Senderempfängerelement zu empfangen und an dieses zu senden, mindestens eine digitale Kamera und mindestens einen ersten Prozessor. Bei der Kamera kann es sich dabei um Kameras in Smartphones, um Kamerasysteme mit CCD bzw. um CMOS Sensor Systeme mit Kommunikationsinterface handeln. Der erste Prozessor kann das von der ersten Senderempfängerelement empfangene Signal verarbeiten und ist erfindungsgemäß dazu konfiguriert folgende Schritte durchzuführen:
- Eine erste Abbildung der Wunde, welche durch die Kamera aufgenommen wurde, zu segmentieren (die erste Abbildung wird in Abwesenheit der Wundauflage aufgenommen). Unter Segmentierung wird hierbei die Unterteilung der Abbildung in mindestens zwei Bereiche nach mindestens einem Kriterium verstanden. Bevorzugt überschneiden sich diese Bereiche nicht.
- Eine zweite Abbildung, welche durch die Kamera aufgenommen wurde und die Wunde und eine auf die Wunde aufgebrachte Wundauflage mit der ersten Abbildung der Wunde zu korrelieren und die Lage der Wundauflage relativ zur Fläche der Wunde zu bestimmen (die zweite Abbildung wird somit aufgenommen, nachdem die Wundauflage auf die Wunde appliziert wurde).
- Diejenigen unabhängig aktivierbaren Bereiche der Wundauflage zu ermitteln, welche über der Fläche der Wunde liegen,
- Die unabhängig aktivierbaren Bereiche der Wundauflage, welche über der Fläche der Wunde liegen, zu aktivieren. Die Anweisung zur Aktivierung oder selektiven Aktivierung wird von dem Bildverarbeitungsmittel zur Wundauflage mittels des zweiten Senderempfängerelements und des ersten Senderempfängerelements übertragen.

Unter "Korrelation" oder "korrelieren" wird verstanden, dass visuelle Merkmale von einer ersten Abbildung mit visuellen Merkmalen auf einer zweiten Abbildung in Verbindung gebracht werden. Hierbei werden visuelle Merkmale der ersten Abbildung eindeutig visuellen Merkmalen auf der zweiten Abbildung zugeordnet. Bevorzugt sind solche visuellen Merkmale einzelne Orientierungspunkte, die zuvor in der ersten Abbildung bestimmt wurden.

Bevorzugt umfasst das Bildaufnahmemittel eine Speichereinheit.

Umfasst die unabhängig aktivierbaren Bereiche der Wundauflage Sensoren wie Photosensoren oder piezoelektrische Ultraschallsensoren, so können die von den Sensoren aufgenommenen Daten an das zweite Senderempfängerelement des Bildaufnahmemittels geschickt werden. In einer bevorzugten Ausführungsform umfasst das Bildaufnahmemittel ein Ausgabemittel. Bei dem Ausgabemittel kann es sich um einen Bildschirm handeln. Auf dem Ausgabemittel kann eine graphische Repräsentation der Daten wiedergegeben werden, die Rückschluss auf den Heilungsverlauf der Wunde gibt und damit der Beobachtung des Wundheilverlaufs dienen kann. Die graphische Repräsentation der Daten wird über den ersten Prozessor berechnet. Auch weitere Eigenschaften der Wunde können mit Hilfe des ersten Prozessors bestimmt und dokumentiert werden. Diese Eigenschaften der Wunde umfassen bevorzugt die Wundgröße, die Wundtiefe und/oder die Zusammensetzung des Gewebes. In einer anderen bevorzugten Ausführungsform umfassen die unabhängig aktivierbaren Bereiche der Wundauflage neben den Sensoren zusätzlich Behandlungselemente, die als Lichtleiter, Photodioden und Ultraschallemitter realisiert sein können. Die von den Sensoren aufgenommenen Daten werden an das erste Senderempfängerelement des Bildaufnahmemittels geschickt. Der im Bildaufnahmemittel enthaltene erste Prozessor bestimmt den aktuellen Zustand der Wunde und speichert den aktuellen Zustand der Wunde in der Speichereinheit ab. Wurde mindestens ein Zustand der Wunde auf der Speichereinheit abgespeichert, so wird der aktuelle Zustand der Wunde mit mindestens einem der vorangegangenen Zustände der Wunde mittels des ersten Prozessors verglichen. Die Aktivierung der Behandlungselemente der unabhängig aktivierbaren Bereiche der Wundauflage kann abhängig vom aktuellen Zustand der Wunde angepasst werden. Die Anpassung an den aktuellen Zustand der Wunde kann dabei beispielsweise eine Verringerung der Anzahl der aktiven Behandlungselemente der unabhängig aktivierbaren Bereiche oder eine Änderung der Frequenz im Falle der Verwendung von Photodioden umfassen. Die Aktivierung der Behandlungselemente der unabhängig aktivierbaren Bereiche wird über das zweite Senderempfängerelement an das erste Senderempfängerelement übermittelt.

Mittels des Bildaufnahmemittels wird die erste Abbildung der Wunde und der unmittelbaren Wundumgebung aufgenommen. Die erste Abbildung muss demnach die die Wunde umgebenden Hautbereiche umfassen. Die erste Abbildung der Wunde wird segmentiert. Zur Segmentierung der Abbildung der Wunde werden bevorzugt Differenzen zwischen zwei Parameterwerten berechnet. Die Parameterwerte umfassen bevorzugt Helligkeitswerte. Die Differenzen zwischen zwei Parameterwerten werden, im Falle von Helligkeitswerten, bevorzugt zwischen den Parameterwerten eines Pixels und zwischen den Parameterwerten seines nächsten Nachbars berechnet. Überschreitet die Differenz beispielsweise einen Grenzwert, so wird eine Grenze eines Segments angenommen. Ein Beispiel für einen Algorithmus, der auf der Differenz zwischen zwei Parameterwerten basiert, kann ein Single-Shot Detector (SSD) sein. In einer anderen bevorzugten Ausführungsform werden bei der Segmentierung Mustererkennungsalgorithmen verwendet. Visuelle Merkmale von Objekten werden verwendet, um ein Bild in mehrere Segmente zu unterteilen. Mögliche Algorithmen können k-Means Clustering, Schwellenwertverfahren oder neuronale Netze, insbesondere Convolutional Neural Networks, umfassen.

Mittels des Bildaufnahmemittels wird die zweite Abbildung der Wunde aufgenommen, sobald die Wundauflage aufgebracht wurde. Die zweite Abbildung umfasst die aufgebrachte Wundauflage ebenso wie Bereiche der Wunde, falls die Wunde nicht von der Wundauflage verdeckt ist. Auch bei der zweiten Abbildung sollte wie bei der ersten Abbildung die unmittelbaren Wundumgebung in der Aufnahme enthalten sein. Vorzugsweise wird für die erste und die zweite Abbildung ein möglichst ähnlicher Bildausschnitt angestrebt. Idealerweise wird die Aufnahme der zweiten Abbildung aus der gleichen Entfernung zur Wunde und dem gleichen Winkel zur Wunde wie die Aufnahme der ersten Abbildung durchgeführt.

Um die Lage der Wundauflage relativ zur Wunde zu bestimmen, wird die erste Abbildung mit der zweiten Abbildung korreliert. In einer bevorzugten Ausführungsform erfolgt die Korrelation der ersten und der zweiten Abbildung mittels Orientierungspunkten. Bei den Orientierungspunkten kann es sich um beispielsweise um Naevi, Umrisse des Körperteils, das die Wunde trägt, Narben, Hautdefekte, Behaarung, auf die unversehrte Haut aufgebrachte Markierungen oder neben der Wunde platzierte Markierungsmittel handeln. Mindestens drei der drei oder mehr Orientierungspunkte müssen sowohl auf der ersten Abbildung als auch auf der zweiten Abbildung zu sehen sein. Bevorzugt sind unsymmetrische Orientierungspunkte, die sowohl auf der ersten Abbildung als auch auf der zweiten Abbildung vollständig zu sehen sind.

Die Lage der Orientierungspunkte zueinander wird in der ersten und zweiten Abbildung bestimmt. Die Lage der Orientierungspunkte zueinander kann über den Abstand der Orientierungspunkte oder eine Kombination aus Winkeln zwischen den Verbindungslinien der Orientierungspunkte und dem Abstand der Orientierungspunkte bestimmt werden. Die Änderung der Lage der Orientierungspunkte zueinander gibt Rückschluss über den Aufnahmewinkel und/oder den Abstand, in dem die Aufnahme der ersten Abbildung zu der Aufnahme der zweiten Abbildung erfolgt ist. Auch die Verzerrung der Orientierungspunkte selbst kann Rückschlüsse über den Aufnahmewinkel oder den Abstand geben. Die Position der Wundauflage kann relativ zu den Orientierungspunkten bestimmt werden. In einer anderen bevorzugten Ausführungsform werden auf Autokorrelationen basierende Algorithmen verwendet, um die Lage der Wundauflage auf dem Patienten anhand der ersten Abbildung und der zweiten Abbildung zu bestimmen.

Bevorzugt ist auf die Abdeckschicht mindestens eine sichtbare Kennzeichnung aufgebracht. Die sichtbare Kennzeichnung kann die Ermittlung der genauen Position der Wundauflage erleichtern, da die Geometrie und die Größe der sichtbaren Kennzeichnung bekannt sind. Eine Verzerrung einer bekannten Geometrie mit definierter Größe und mindestens einer symmetriebrechenden Eigenschaft kann Rückschlüsse über eine Abmessung oder einen Aufnahmewinkel erlauben. Die sichtbare Kennzeichnung besteht aus mindestens drei Punkten. Ebenfalls kann die sichtbare Kennzeichnung Muster, Schrift, Logos und/oder Bilder umfassen.

Die Position der Wundauflage relativ zu den Orientierungspunkten und damit die Position der Wundauflage relativ zu der Position des segmentierten Wundabbildung wird bestimmt. Über die sichtbaren Kennzeichnungen auf der Wundauflage kann die absolute Größe der auf der ersten und zweiten Abbildung dargestellten Objekte, insbesondere der Wunde, ermittelt werden.

Die unabhängig aktivierbaren Bereiche, die über der Wunde liegen, werden einem Bereich der zu behandelnden Wunde zugeordnet. Vorzugsweise werden nur diejenigen unabhängig aktivierbaren Bereiche der Wundauflage, die vollständig über der Wunde liegen, aktiviert.

In einer anderen bevorzugten Ausführungsform wird ein unabhängig aktivierbarer Bereich nur dann aktiviert, wenn eine vorgegebene Bedingung erfüllt ist. Bei der vorgegebenen Bedingung kann es sich um die Freigabe durch einen Anwender oder durch eine dritte Person handeln. Alternativ oder zusätzlich kann es sich bei der vorgegebenen Bedingung um einen oder mehrere Werte eines oder mehrerer Parameter, welche die Eigenschaft der Wunde charakterisieren, handeln. Der Parameter kann einen Lesewert umfassen. Der Parameter kann auch eine positive Identifikation (d.h. eine erfolgreiche Erkennung) des Gewebetyps bzw. einer Wundkondition im Areal umfassen.

Die unabhängig aktivierbaren Bereiche der Wundauflage, welche über der Fläche der Wunde liegen, werden aktiviert. Das Behandlungselement oder die Behandlungselement können somit selektiv aktiviert werden, wobei der therapeutische Effekt auf die eigentliche Wundfläche beschränkt bleibt. Ebenso ist es unter Verwendung der vorliegenden Erfindung möglich, ausgewählte Bereiche innerhalb der Wunde selektiv zu behandeln.

### Abbildungen

Zum besseren Verständnis der vorliegenden Erfindung und ihrer Vorteile wird nun auf die folgenden Ausführungsformen in Verbindung mit den zugehörigen Abbildungen verwiesen. Im Folgenden wird die Erfindung anhand exemplarischer Ausführungsformen, die in den schematischen Abbildungen angegeben sind, näher erläutert.

### Abbildung 1

Schematische Darstellung einer Ausführungsform einer mit der vorliegenden Patentanmeldung vorgeschlagenen Wundauflage, welche dem Anwender erlaubt, innerhalb einer zu behandelnden Wunde einen therapeutischen Effekt selektiv nur auf einen Teilbereich der zu behandelnden Wunde auszuüben oder innerhalb einer Wundstelle nur auf die eigentliche Wunde einen therapeutischen Effekt auszuüben. Es wird ein Schnitt durch die Wundauflage dargestellt.

### Abbildung 2

Schematische Darstellung einer Ausführungsform des Behandlungselements in der Aufsicht.

### Abbildung 3

Schematische Darstellung eines Verfahrens zur selektiven Behandlung eines Wundbereichs.

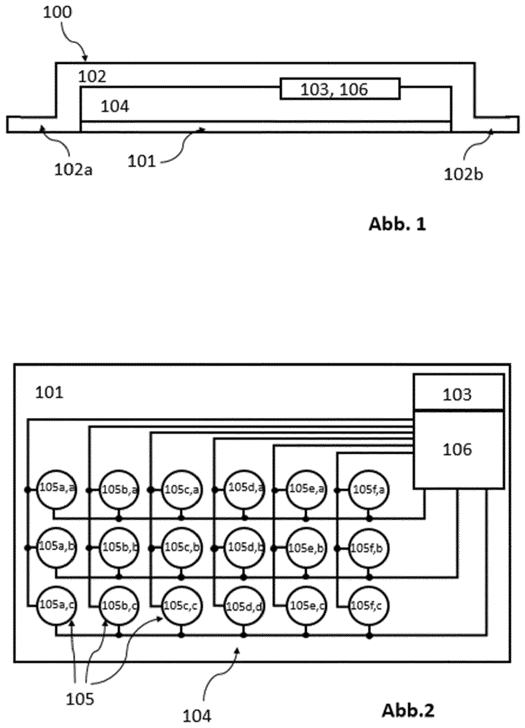

Abbildung 1 zeigt eine bevorzugte Ausführungsform der Wundauflage 100. Die Wundauflage 100 umfasst eine Wundkontaktschicht 101, eine Abdeckschicht 102 und ein Behandlungselement 104, wobei das Behandlungselement 102 zwischen Wundkontaktschicht 101 und Abdeckschicht 102 angeordnet ist. Die Wundkontaktschicht 101 liegt bei Gebrauch der Wundauflage 100 direkt auf der Wundstelle oder der Wunde des Patienten auf. Die Abdeckschicht 102 kann adhäsive Bereiche 102a, 102b auf der dem Patienten zugewandten Seite der Abdeckschicht aufweisen. Die adhäsiven Bereiche 102a, 102b dienen der Befestigung der Wundauflage 100.

Die Wundauflage 100 umfasst darüber hinaus ein erstes Senderempfängerelement 103 und eine Stromquelle 106. Die Stromquelle 106 versorgt das Behandlungselement 104 und das erste Senderempfängerelement 103 mit Energie. Bei der Stromquelle 106 kann es sich um eine Batterie handeln. Alternativ kann die Stromquelle 106 Energie mittels Energy Harvesting bereitstellen.

Abbildung 2 zeigt eine mögliche Ausführungsform des Behandlungselements 104 der Wundauflage 100. Das Behandlungselement umfasst unabhängig aktivierbare Bereiche. Die unabhängig aktivierbaren Bereiche 105 sind bevorzugt in einer Ebene parallel zur Wundkontaktschicht 101 angeordnet.

Jeder unabhängig aktivierbare Bereich 105 hat keine gemeinsame Fläche mit seinen benachbarten Bereichen und kann auch einzeln aktiviert werden. Die unabhängig aktivierbaren Bereiche 105 umfassen elektronische Bauteile, die in einer matrixförmigen Anordnung auf einem flexiblen Substrat wie beispielsweise Silikon aufgebracht oder eingebettet sind. Die elektronischen Bauteile der unabhängig aktivierbaren Bereiche können Lichtleiter, Photodioden, Photosensoren, piezoelektrische Ultraschallemitter und piezoelektrische Ultraschallsensoren sein.

Die elektronischen Bauteile 105a,a bis 105f,c sind so geschaltet, dass sie einzeln aktiviert werden können.

Mittels der Stromquelle 106 werden die elektronischen Bauteile 105a,a bis 105f,c mit Energie versorgt. Das erste Senderempfängerelement 103 ist ebenfalls an die Stromquelle 106 gekoppelt. Mittels des ersten Senderempfängerelements 103 werden Daten, die den Zustand der Wunde betreffen an ein zweites Senderempfängerelement (nicht dargestellt) gesendet. Ebenso kann das erste Senderempfängerelement 103 Anweisungen zur Aktivierung der unabhängig aktivierbaren Bereiche 105 über das zweite Senderempfängerelement empfangen.

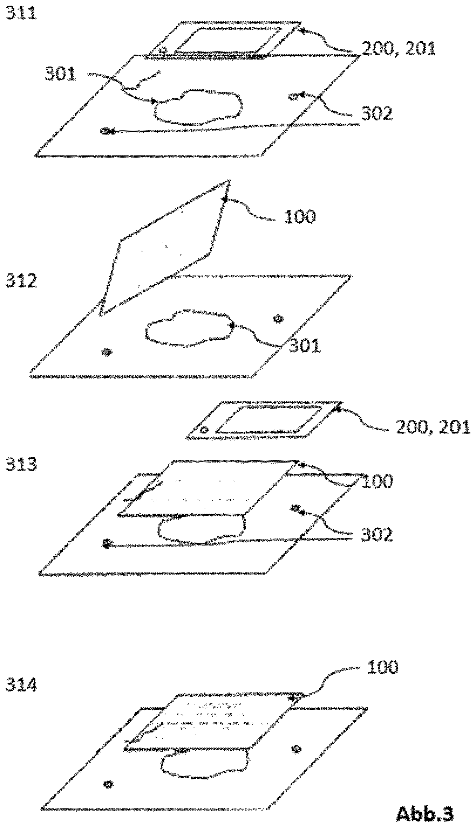

Abbildung 3 zeigt Schritt für Schritt (Abfolge 311 - 314) beispielhaft, wie das Kit aus Wundauflage 100 und Bildaufnahmemittel 200 verwendet werden kann:
Im Schritt 311 wird eine erste Abbildung der Wunde 301 mittels der Kamera 201 im Bildaufnahmemittel 200 aufgenommen. Bei dem Bildaufnahmemittel 200 kann es sich um ein bluetooth-fähiges Smartphone mit Kamera handeln. Die erste Abbildung der Wunde 301 wird mittels eines digitalen Verfahrens segmentiert. Die optischen Orientierungspunkte 302 werden bestimmt. Die Orientierungspunkte sind beispielsweise Naevi, aufgebrachte Marker oder Umrisse des Körperteils, auf dem sich die Wunde befindet.

In Schritt 312 wird die Wundauflage 100 auf die Wunde 301 aufgebracht.

In Schritt 313 wird eine zweite Abbildung der Wunde 301 mit der in Schritt 312 applizierten Wundauflage 100 durch die Kamera 201 aufgenommen. Die zweite Abbildung der Wunde wird mit der ersten Abbildung der Wunde korreliert. Die Position der Wundauflage 100 relativ zur Fläche der Wunde wird mit Hilfe der Orientierungspunkte 302 bestimmt.

Die unabhängig aktivierbaren Bereiche der Wundauflage 100 werden mit der Fläche der Wunde mittels der ersten Abbildung und der zweiten Abbildung, sowie unter Zuhilfenahme der Orientierungspunkte 302, korreliert.

Auf die Wundstelle können sodann zusätzliche Abdeckungen oder zusätzliches Verbandmaterial aufgebracht werden.

In Schritt 314 werden die unabhängig aktivierbaren Bereiche der Wundauflage 100, die eine vorgegebene Bedingung erfüllen, aktiviert. Die Bedingung kann umfassen, dass die unabhängig aktivierbaren Bereiche mindestens zu 100% über der Fläche der Wunde 301 liegen.

## Patentansprüche

1. Vorrichtung umfassend eine Wundauflage (100) und ein Bildaufnahmemittel (200), wobei die Wundauflage (100)
a. eine bei Gebrauch der Wundauflage (100) wundabseitig angeordnete Abdeckschicht,
b. ein erstes Senderempfängerelement (103), welches Daten übertragen und empfangen kann,
umfasst,
und wobei das Bildaufnahmemittel (200)
a. eine digitale Kamera,
b. einen ersten Prozessor,
c. ein zweites Senderempfängerelement, welches dazu ausgestaltet ist Daten von dem ersten Senderempfängerelement (103) zu empfangen und an das erste Senderempfängerelement (103) zu senden,
umfasst,
wobei die Wundauflage (100) ein Behandlungselement (104) umfasst, welches mindestens zwei unabhängig aktivierbare Bereiche aufweist,
wobei jeder unabhängig aktivierbare Bereich keine gemeinsame Fläche mit seinen benachbarten Bereichen hat und einzeln aktiviert werden kann,
so dass entweder innerhalb einer zu behandelnden Wunde ein therapeutischer Effekt nur auf einen Teilbereich der zu behandelnden Wunde ausgeübt werden kann oder dass innerhalb einer Wundstelle, welche die zu behandelnde Wunde und die die Wunde umgebende Haut umfasst, nur auf die Wunde ein therapeutischer Effekt ausgeübt werden kann,
**dadurch gekennzeichnet, dass** der erste Prozessor dazu konfiguriert ist,
- eine erste Abbildung, die eine Abbildung der zu behandelnden Wunde umfasst und welche durch das Bildaufnahmemittel (200) aufgenommen wurde, zu segmentieren, so dass die erste Abbildung in ein oder mehrere Segmente unterteilt wird und die ein oder mehreren Segmente der ersten Abbildung, die die zu behandelnde Wunde darstellen, als Fläche der Wunde gekennzeichnet werden,
- eine zweite Abbildung, die eine Abbildung der Wundauflage (100) auf der zu behandelnden Wunde umfasst und welche durch das Bildaufnahmemittel (200) aufgenommen wurde, mit der ersten Abbildung zu korrelieren, so dass die Position der Wundauflage (100) relativ zur Fläche der Wunde bestimmt wird,
wobei die Korrelation visuelle Merkmale umfasst und die visuellen Merkmale der ersten Abbildung eindeutig visuellen Merkmalen auf der zweiten Abbildung zugeordnet werden
- diejenigen unabhängig aktivierbaren Bereiche der Wundauflage (100) zu ermitteln, welche über der Fläche der Wunde liegen,
- die unabhängig aktivierbaren Bereiche der Wundauflage (100), welche über der Fläche der Wunde liegen, zu aktivieren oder selektiv zu aktivieren,
wobei die Anweisung zur Aktivierung oder selektiven Aktivierung mittels des zweiten Senderempfängerelements und des ersten Senderempfängerelements von dem Bildverarbeitungsmittel zur Wundauflage (100) übertragen wird.

2. Vorrichtung nach Anspruch 1, wobei das Behandlungselement (104) bei Gebrauch der Wundauflage (100) in direktem Kontakt mit dem Wundgrund ist.

3. Vorrichtung nach Anspruch 1, wobei die Wundauflage (100) eine Wundkontaktschicht (101) umfasst, die bei Gebrauch der Wundauflage (100) wundseitig angeordnet ist.

4. Vorrichtung nach Anspruch 3, wobei das Behandlungselement (104) zwischen der Wundkontaktschicht (101) und der Abdeckschicht (102) angeordnet ist.

5. Vorrichtung nach Anspruch mindestens einem der vorgenannten Ansprüche, wobei die mindestens zwei unabhängig aktivierbaren Bereiche in einer zum Wundgrund parallelen Ebene angeordnet sind.

6. Vorrichtung nach mindestens einem der vorgenannten Ansprüche, wobei die mindestens zwei unabhängig aktivierbaren Bereiche jeweils mindestens einen der Bauteile ausgewählt aus einer Photodiode, einem Lichtleiter, oder einem piezoelektrische Ultraschallemitter umfassen.

7. Vorrichtung nach mindestens einem der vorgenannten Ansprüche, wobei die Daten kontaktlos durch das erstes Senderempfängerelement (103) an das zweite Senderempfängerelement übertragen und empfangen werden können.

8. Vorrichtung nach mindestens einem der vorgenannten Ansprüche, wobei die Wundauflage (100) mindestens eine sichtbare Kennzeichnung auf der wundabgewandten Seite der Abdeckschicht (102) umfasst.

9. Vorrichtung nach mindestens einem der vorgenannten Ansprüche, wobei die Wundauflage (100) einen zweiten Prozessor umfasst.

10. Vorrichtung nach mindestens einem der vorgenannten Ansprüche, wobei eine Anzahl der mindestens zwei unabhängig aktivierbaren Bereiche der Wundauflage (100) aktiviert oder selektiv aktiviert werden, wenn mindestens eine zusätzliche vorgegebene Bedingung erfüllt ist.

11. Vorrichtung nach Anspruch 10, wobei die vorgegebene Bedingung eine Freigabe durch einen Anwender oder einer dritten Person und/oder ein Wert eines Parameters, der die Eigenschaft der Wunde charakterisiert, ist.

12. Vorrichtung nach mindestens einem der Ansprüche 8 bis 11, wobei die Wundauflage (100) mindestens eine sichtbare Kennzeichnung nach Anspruch 8 umfasst und wobei diejenigen unabhängig aktivierbaren Bereiche der Wundauflage (100), welche über der Fläche der Wunde liegen, mittels der mindestens einen sichtbaren Kennzeichnung ermittelt werden.

## Claims

1. Device comprising a wound dressing (100) and an image recording means (200),
wherein the wound dressing (100) comprises
a. a cover layer arranged distant from the wound when the wound dressing (100) is used,
b. a first transceiver element (103) capable of transmitting and receiving data,
and wherein the image recording means (200) comprises
a. a digital camera,
b. a first processor,
c. a second transceiver element configured to receive data from the first transceiver element (103) and transmit data to the first transceiver element (103),
wherein the wound dressing (100) comprises a treatment element (104) which has at least two independently activatable regions,
wherein each independently activatable region shares no common area with its adjacent regions and can be activated on an individual basis,
such that, either within a wound to be treated, a therapeutic effect can be exerted only on a portion of the wound to be treated or that, within a wound site comprising the wound to be treated and the skin surrounding the wound, a therapeutic effect can be exerted only on the wound,
**characterized in that** the first processor is configured
- to segment a first image, which comprises an image of the wound to be treated and which was recorded by the image recording means (200), such that the first image is divided into one or more segments and the one or more segments of the first image which represent the wound to be treated are labelled as area of the wound,
- to correlate a second image, which comprises an image of the wound dressing (100) on the wound to be treated and which was recorded by the image recording means (200), with the first image such that the position of the wound dressing (100) is determined relative to the area of the wound,
wherein the correlation comprises visual features and the visual features of the first image are uniquely assigned to visual features on the second image,
- to ascertain those independently activatable regions of the wound dressing (100) which are located over the area of the wound,
- to activate or selectively activate the independently activatable regions of the wound dressing (100) which are located over the area of the wound,
wherein the instruction for activation or selective activation is transmitted from the image processing means to the wound dressing (100) by means of the second transceiver element and the first transceiver element.

2. Device according to Claim 1, wherein the treatment element (104) is in direct contact with the wound bed when the wound dressing (100) is used.

3. Device according to Claim 1, wherein the wound dressing (100) comprises a wound contact layer (101) arranged on the side facing the wound when the wound dressing (100) is used.

4. Device according to Claim 3, wherein the treatment element (104) is arranged between the wound contact layer (101) and the cover layer (102).

5. Device according to at least one of the preceding claims, wherein the at least two independently activatable regions are arranged in a plane parallel to the wound bed.

6. Device according to at least one of the preceding claims, wherein the at least two independently activatable regions each comprise at least one of the components selected from a photodiode, a light guide or a piezoelectric ultrasonic emitter.

7. Device according to at least one of the preceding claims, wherein the data, by means of the first transceiver element (103), can be transmitted to and received contactlessly by the second transceiver element.

8. Device according to at least one of the preceding claims, wherein the wound dressing (100) comprises at least one visible label on the side of the cover layer (102) facing away from the wound.

9. Device according to at least one of the preceding claims, wherein the wound dressing (100) comprises a second processor.

10. Device according at least one of the preceding claims, wherein a number of the at least two independently activatable regions of the wound dressing (100) are activated or selectively activated if at least one additional specified condition is satisfied.

11. Device according to Claim 10, wherein the specified condition is an enablement by a user or a third person and/or a value of a parameter characterizing the property of the wound.

12. Device according to at least one of Claims 8 to 11, wherein the wound dressing (100) comprises at least one visible label according to Claim 8 and wherein those independently activatable regions of the wound dressing (100) located over the area of the wound are ascertained by means of at least one visible label.

## Revendications

1. Dispositif comprenant un pansement (100) et un moyen d'acquisition d'images (200), le pansement (100) comprenant
a. une couche de recouvrement disposée du côté plaie lorsque le pansement (100) est utilisé,
b. un premier élément émetteur-récepteur (103) qui peut transmettre et recevoir des données,
et les moyens d'acquisition d'images (200) comprenant
a. un appareil photo numérique,
b. un premier processeur,
c. un deuxième élément émetteur-récepteur qui est conçu pour recevoir des données du premier élément émetteur-récepteur (103) et pour les envoyer au premier élément émetteur-récepteur (103),
le pansement (100) comprenant un élément de traitement (104) qui comporte au moins deux zones activables indépendamment,
chaque zone activable indépendamment ne comportant pas de surface commune avec ses zones voisines et pouvant être activée individuellement de sorte que soit à l'intérieur d'une plaie à traiter un effet thérapeutique ne puisse être exercé que sur une souszone de la plaie à traiter soit à l'intérieur d'un site de plaie, qui comprend la plaie à traiter et la peau entourant la plaie, un effet thérapeutique ne puisse être exercé que sur la plaie,
**caractérisé en ce que** le premier processeur est configuré pour
- segmenter une première image, qui comprend une image de la plaie à traiter et qui a été acquise par le biais du moyen d'acquisition d'image (200) de sorte que la première image soit divisée en un ou plusieurs segments et les un ou plusieurs segments de la première image qui représentent la plaie à traiter, soient caractérisés comme surface de la plaie,
- corréler une deuxième image, qui comprend une image du pansement (100) sur la plaie à traiter et qui a été acquise par le biais du moyen d'acquisition d'image (200), avec la première image de façon à déterminer la position du pansement (100) par rapport à la surface de la plaie,
la corrélation comprenant des caractéristiques visuelles et les caractéristiques visuelles de la première image étant associées de manière unique aux caractéristiques visuelles de la deuxième image,
- déterminer les zones, activables indépendamment, du pansement (100) qui sont situées au-dessus de la surface de la plaie,
- pour activer ou activer sélectivement les zones activables indépendamment du pansement (100) qui sont situées au-dessus de la surface de la plaie, l'instruction d'activation ou d'activation sélective étant transmise du moyen de traitement d'image au pansement (100) à l'aide du deuxième élément émetteur-récepteur et du premier élément émetteur-récepteur.

2. Dispositif selon la revendication 1, l'élément de traitement (104) étant en contact direct avec la base de la plaie lorsque le pansement (100) est utilisé.

3. Dispositif selon la revendication 1, le pansement (100) comprenant une couche de contact avec la plaie (101) qui est disposée côté plaie lorsque le pansement (100) est utilisé.

4. Dispositif selon la revendication 3, l'élément de traitement (104) étant disposé entre la couche de contact avec la plaie (101) et la couche de recouvrement (102).

5. Dispositif selon l'une au moins des revendications précédentes, les au moins deux zones activables indépendamment étant disposées dans un plan parallèle à la base de la plaie.

6. Dispositif selon l'une au moins des revendications précédentes, les au moins deux zones activables indépendamment comprenant chacune au moins un des composants choisis parmi une photodiode, un guide de lumière ou un émetteur d'ultrasons piézoélectrique.

7. Dispositif selon l'une au moins des revendications précédentes, les données pouvant être transmises par le premier élément émetteur-récepteur (103) au deuxième élément émetteur-récepteur, et reçues par celui-ci, sans contact.

8. Dispositif selon l'une au moins des revendications précédentes, le pansement (100) comprenant au moins un marquage visible sur le côté de la couche de recouvrement (102) qui est opposé à la plaie.

9. Dispositif selon l'une au moins des revendications précédentes, le pansement (100) comprenant un deuxième processeur.

10. Dispositif selon l'une au moins des revendications précédentes, un certain nombre des au moins deux zones activables indépendamment du pansement (100) étant activées ou activées sélectivement lorsqu'au moins une condition spécifiée supplémentaire est remplie.

11. Dispositif selon la revendication 10, la condition spécifiée étant une libération par un utilisateur ou un tiers et/ou une valeur d'un paramètre qui caractérise la propriété de la plaie.

12. Dispositif selon l'une au moins des revendications 8 à 11, le pansement (100) comprenant au moins un marquage visible selon la revendication 8 et les zones activables indépendamment du pansement (100) qui sont situées au-dessus de la surface de la plaie étant déterminées au moyen de l'au moins un marquage visible.
